Europäisches Patentamt

European Patent Office

Office européen des brevets

(19)

(11) Veröffentlichungsnummer: **0 255 893 B1**

(12) **EUROPÄISCHE PATENTSCHRIFT**

(45) Veröffentlichungstag der Patentschrift: **13.03.91**

(21) Anmeldenummer: **87110536.7**

(22) Anmeldetag: **21.07.87**

(51) Int. Cl.⁵: **C07C 19/045**, C07C 17/156, B01D 53/04

(54) Verfahren und Vorrichtung zur Rückgewinnung von 1,2-Dichlorethan aus Abgasen.

(30) Priorität: **08.08.86 DE 3626853**

(43) Veröffentlichungstag der Anmeldung:
**17.02.88 Patentblatt 88/07**

(45) Bekanntmachung des Hinweises auf die
Patenterteilung:
**13.03.91 Patentblatt 91/11**

(84) Benannte Vertragsstaaten:
**BE DE ES FR GB IT NL SE**

(56) Entgegenhaltungen:
**DE-A- 2 400 417**
**DE-A- 3 048 649**
**US-A- 4 151 212**

(73) Patentinhaber: **HOECHST AKTIENGESELL-SCHAFT**
**Postfach 80 03 20**
**W-6230 Frankfurt am Main 80(DE)**

(72) Erfinder: **Schuchardt, Kurt**
**Wittelsbacher Strasse 78g**
**W-5040 Brühl(DE)**
Erfinder: **Scholz, Harald, Dr.**
**Am Beissel 8**
**W-5042 Erftstadt(DE)**
Erfinder: **Niehus, Erich**
**Kapellenstrasse 21**
**W-5303 Bornheim(DE)**
Erfinder: **Adam, Harald**
**Am Lindenfeld 7**
**W-5042 Erftstadt(DE)**

**Beschreibung**

Die Erfindung betrifft ein kontinuierliches Verfahren und eine Vorrichtung zur vollständigen Entfernung und Rückgewinnung von 1,2-Dichlorethan aus den bei der Herstellung von 1,2-Dichlorethan durch Oxichlorierung von Ethylen mit Chlorwasserstoff und Sauerstoff anfallenden Abgasen, welche über Aktivkohle geleitet und anschließend bei Temperaturen oberhalb 900° C verbrannt sowie schließlich von Chlorwasserstoff und Chlor gereinigt werden, wobei die Aktivkohle mit heißem Wasserdampf von 1,2-Dichlorethan befreit und regeneriert wird.

Die Oxichlorierung von Ethylen wird in großtechnishem Maßstab zur Gewinnung von 1,2-Dichlorethan durchgeführt. Dabei werden die Einsatzstoffe Ethylen, Chlorwasserstoffgas und molekularer Sauerstoff etwa im stöchiometrischen Verhältnis (1 : 2 : 0,5), jedoch bevorzugt mit einem Überschuß von Ethylen und Sauerstoff (1 : 1,9 : 0,6), in der Gasphase an einem Deacon-Katalysator (z.B. $CuCl_2$ auf $Al_2O_3$-Träger) bei erhöhter Temperatur (z.B. 200 - 250° C) und erhöhtem Druck (z.B. 1,7 - 5,0 bar) zu 1,2-Dichlorethan umgesetzt. Die Reaktion kann in einem Fest- oder Fließbett-Reaktor erfolgen. Moderne Oxichlorierungsverfahren arbeiten mit reinem Sauerstoff statt mit Luft, um die Bewältigung großer Abgasmengen zu vermeiden. Genannt seien hier die Verfahren der DE 27 18 878 C3 und 27 42 409 C3. Bei Verwendung reinen oder hochprozentigen Sauerstoffs betragen die Abgasmengen nur 2 bis 10 % (etwa 16 bis 80 $m^3$/t 1,2-Dichlorethan) der bei Verwendung von Luft üblichen Abgasmengen (etwa 800 $m^3$ je Tonne 1,2-Dichlorethan, gemessen unter Normalbedingungen).

Nach Verlassen der Reaktionszone wird das Gas, das u.a. 0,1 - 1,0 Vol% $C_2H_4$ und 4 - 8 Vol% $O_2$ enthält, zuerst durch Einspritzen von Wasser auf 80 bis 100° C, sodann indirekt mit Wasser auf 35 bis 40° C und schließlich mit Kühlsole auf 1 bis 10° C in mehreren Stufen abgekühlt. Dabei werden die Reaktionsprodukte, hauptsächlich 1,2-Dichlorethan und Wasser, auskondensiert und der weiteren Verarbeitung zugeführt.

Das Abgas hinter der letzten Kondensationsstufe ist entsprechend dem Druck und der Temperatur mit 1,2-Dichlorethan und Wasserdampf gesättigt. Es enthält nicht-umgesetztes Ethylen und molekularen Sauerstoff entsprechend dem angewendeten Überschuß, ferner Kohlenoxide, die als Nebenprodukte durch Verbrennung von Ethylen entstehen, Stickstoff, der zu Spül- und Meßzwecken diente und schließlich in geringer Konzentration leichtsiedende organische Substanzen.

Dieses Abgas wird komprimiert, mit molekularem Sauerstoff auf 10 bis 30 Vol% $O_2$ angereichert und in den Reaktor zurückgefördert, wo der Sauerstoff wieder mit Ethylen und Chlorwasserstoff zu 1,2-Dichlorethan reagiert. Aus diesem Kreislauf wird an Abgas über eine Regeleinrichtung zur Konstanthaltung des Druckes im Reaktorsystem gerade so viel ausgeschleust, wie an Stickstoff zugeführt und an gasförmigen Nebenprodukten, hauptsächlich Kohlenoxiden, gebildet wird.

Die DE 24 00 417 C3 beschreibt ein Verfahren zur Beseitigung des luftverschmutzenden Abgases bei der großtechnischen Synthese von Dichlorethan durch Oxichlorierung von Ethylen, worin das Abgas nach der Kondensation bei Temperaturen zwischen -30 und +50° C, vorzugsweise zwischen -10 und +10° C, über Aktivkohle geleitet wird, wobei ein Teil der organischen Verunreinigungen adsorbiert wird und die restlichen brennbaren Anteile des Abgases bei Temperaturen zwischen 500 und 2000° C verbrannt werden.

Bei diesem Verfahen wird der Sauerstoff jedoch, wie u.a. die Gasanalysen in den dortigen Beispielen 1 und 2 zeigen (geringe CO und $CO_2$-Konzentration infolge der starken Verdünnung durch Luftstickstoff), bevorzugt in Form von Luft eingesetzt. Die daraus resultierenden großen Abgasmengen machen das Verfahren wegen der erforderlichen Dimensionierung der Apparatur und der hohen Energiekosten für die Regenerierung unwirtschaftlich und gegenüber herkömmlichen Verfahren auf der Basis der Absorption mit organischen Lösemitteln, z.B. Kerosin, nicht konkurrenzfähig.

Beim Verfahren der DE 24 00 417 C3 wird das Abgas vermutlich mit der Temperatur, mit der es die letzte Kondensationsstufe verläßt, über die Aktivkohle geleitet. Beim Beispiel 1 sind dies -5° C, was zur Folge hat, daß das Dichlorethan nur zu etwa 90 % adsorbiert wird. Der im Abgas enthaltene Wasserdampf wird bei Temperaturen in der Nähe des Taupunkts von Wasser gleichzeitig mit 1,2-Dichlorethan von der Aktivkohle adsorbiert und setzt die Adsorptionsfähigkeit der Aktivkohle für organische Stoffe, hier insbesondere 1,2-Dichlorethan, herab.

Außerdem führt die Abkühlung wasserdampfgesättigter Gase auf Temperaturen unter 0° C zur Bildung von Eis, das die Kondensatoren innerhalb kurzer Zeit verstopft und die Fortführung des Prozesses unmöglich macht.

Es wurde nun überraschenderweise gefunden, daß bei einem mit im wesentlichen reinem Sauerstoff arbeitenden Oxichlorierungsverfahren das 1,2-Dichlorethan vollständig aus dem ausgeschleusten Abgas zurückgewonnen werden kann, wenn man das mit 1,2-Dichlorethan beladene, eine Temperatur von 1° bis 10° C aufweisende Abgas auf einen Temperatur, die mindestens 20° C oberhalb des jeweiligen Taupunktes für Wasser liegt, erwärmt und über die Aktivkohle leitet, welche in zwei gleichartigen Zonen angeordnet ist, wovon in einem Zeitraum von 8 bis 16 Stunden jeweils eine Zone 1,2-Di-chlorethan aus dem Abgas adsorbiert, während die andere Zone in drei Stufen regeneriert wird, derart , daß man die mit 1,2-Dichlorethan beschickte Aktivkohle-Zone in der ersten Regenerierungsstufe mit Wasserdampf von 4 bis 10 bar beaufschlagt und von 1,2-Dichlorethan befreit, in der zweiten Regenerierungsstufe mit 1,2-dichlorethanfreiem und auf 100 bis 150°C erhitztem Abgas trocknet, in der dritten Regenerierungsstufe mit 1,2-dichlorethanfreiem Abgas kaltbläst, kondensiertes 1,2-Dichlorethan und Dampfkondensat in die Oxichlorierung zurückführt und das Abgas der Verbrennung zuführt.

Darüberhinaus kann das Verfahren der Erfindung bevorzugt und wahlweise dadurch gekennzeichnet sein, daß

a) man das mit 1,2-Dichlorethan beladene Abgas auf 25 bis 35°C erwärmt;

b) man eine Aktivkohle mit einer aktiven Oberfläche nach BET von mindestens 1200 m²/g einsetzt;

c) die Verweilzeit des Abgases in der 1,2-Dichlorethan adsorbierenden Aktivkohle-Zone mindestens 10, vorzugsweise 20 bis 60 Sekunden, beträgt;

d) das Umschalten von einer Regenerierungsstufe auf die nächste und von einer auf die andere Aktivkohle-Zone vollautomatisch von einem Zeitprogramm gesteuert wird.

Die Erfindung betrifft weiterhin eine Vorrichtung zur kontinuierlichen Durchführung des erfindungsgemäßen Verfahrens, welches gekennzeichnet ist durch 2 gleichartige, senkrecht angeordnete, jeweils oben und unten durch Verbindungsleitungen überbrückte, mit Aktivkohle gefüllte zylindrische Adsorber, deren Verhältnis Höhe zu Durchmesser 5:1 bis 10:1 beträgt, wobei die Adsorptionskapazität der Aktivkohlemenge eines Adsorbers zeitlich ausreicht für die Regenerierung der gleichen Aktivkohlemenge im anderen Adsorber; je 1 -Vierwegeventil in den beiden Verbindungsleitungen, eine erste Zu-führrleitung für 1,2-dichlorethanhaltiges Abgas von der Oxichlorierungsanlage zum unteen Vierwegeventil, eine Heizung in der ersten Zuführrleitung; eine erste Abzugsleitung für 1,2-dichlorethanfreies Abgas vom oberen Vierwegeventil zu einem Verbrennungsofen; eine zweite Zuführrleitung mit Absperrventil zum oberen Vierwegeventil zwecks Transport von Heißdampf für die abwechselnde Desorption der Aktivkohle sowie von 1,2-dichlorethanfreiem Abgas zum Trocknen oder Kaltblasen der desorbierten Aktivkohle; eine dritte Verbindungsleitung für 1,2-dichlorethanfreies Abgas mit Wärmetauscher zwischen der ersten Abzugsleitung und der zweiten Zuführrleitung; eine zweite Abzugsleitung für 1,2-dichlorethanhaltigen Heißdampf sowie wasserdampfgesättigtes Abgas mit kondensator vom unteren Vierwegeventil zu einem Abscheider für die Abscheidung und gleichzeitige Phasentrennung von kondensiertem 1,2-Dichlorethan und Wasser.

Die Vorrichtung der Erfindung kann weiterhin bevorzugt und wahlweise gekennzeichnet sein durch

e) eine dritte Abzugsleitung für 1,2-dichlorethanfreies Abgas vom Abscheider zum Verbrennungsofen;

f) eine vierte Abzugsleitung für 1,2-Dichlorethan und Kondensat vom Abscheider zur Oxichlorierungsanlage mit Standhaltungsregler für den Abscheider.

Die erfindungsgemäße 3 Stufen-Regenerierung der Aktivkohle unterscheidet sich wesentlich von den Angaben in der DE 24 00 417 C3, in der lediglich eine Regenerierung mit Wasserdampf von etwa 100°C (etwa 1 bar) vorgesehen ist. Demgegenüber sieht das Verfahren der Erfindung in der 1. Stufe eine Regenerierung mit wesentlich heißerem Wasserdampf, nämlich von 4 bis 10 bar, in der 2. Stufe eine Trocknung mit heißem und in der 3. Stufe ein Kaltblasen mit kaltem, 1,2-dichlorethanfreiem Abgas vor. Das zurückgewonnene 1,2-Dichlorethan wird laufend aus dem Abscheider entfernt, damit im Abscheider in der 2. und 3. Regenerierungsstufe keine erneute Resorption von 1,2-Dichlorethan durch das vorher zum Trocknen und Kaltblasen über die Aktivkohle geleitete 1,2-dichlorethanfreie Abgas erfolgt.

Nachfolgend wird das Verfahren und insbesondere die Vorrichtung der Erfindung anhand des beigefügten Fließschemas näher erläutert:

Das aus der Oxichlorierungsanlage (1) über das Druckhalteventil (2) ausgeschleuste Abgas wird in Leitung (3) mit Hilfe der Heizung (4) mindestens 20°C über den Taupunkt für Wasser erwärmt und durch Vierwege-Ventil (5) zum Adsorber (6) geleitet, wo es durch Adsorption an Aktiv-Kohle von 1,2-Dichlorethan befreit wird. Es strömt dann über Vierwege-Ventil (7) und Leitung (8) zur Verbrennungsanlage (9), sofern es nicht zur Regenerierung des Adsorbers (10) benötigt und deshalb vorher durch diesen geleitet wird. Sofort nach dem automatischen Umschalten auf den Adsorber (6) beginnt die Regenerierung der Aktiv-Kohle im Adsorber (10) durch Öffnen des Dampfventils (11). Der Dampf von 4 - 10 bar strömt durch Leitung (12) und Vierwege-Ventil (7) von oben nach unter durch den Adsorber (10) und treibt das 1,2-Dichlorethan aus der Aktiv-Kohle aus. 1,2-Dichlorethan und Dampf gelangen durch Vierwege-Ventil (5) und Leitung (13) in den Kondensator (14), wo beide auskondensieren und in den Abscheider (15) abfließen. Dort erfolgt Phasentrennung, wobei aufgrund der unterschiedlichen Dichten 1,2-Dichlorethan die untere und $H_2O$ die obere Phase bilden. Dadurch wird mittels Pumpe (16) und Standhaltungsregler (17) 1,2-Dichlorethan zuerst und sofort, jedoch gefolgt von einem Teil des Wassers, über Leitung (18) in die Oxichlorierungsanlage (1) zurückgefördert. Im Abscheider (15) befindet sich als stabile Phase immer nur ein Teil des Wassers.

Nach vorgegebener Zeit wird die 1. Regenerierungstufe automatisch beendet und die 2. Regenerierungstufe, d. h. das Trocknen der Aktiv-Kohle, wie folgt eingeleitet: Dampfregelventil (11) schließt; Dampfregelventil (19) öffnet; Regelventil (20) öffnet; Regelventil (21) schließt.

Das im Adsorber (6) vom 1,2-Dichlorethan befreite Abgas wird dadurch umgelenkt und strömt über Leitung (22) auf dem Wege Ventil (20), Wärmetauscher (23), Leitung (12) und Vierwegeventil (7) auf 100 - 150° C erhitzt durch den Adsorber (10) und dann über Vierwege-Ventil (5), Leitung (13), Kondensator (14), Abscheider (15), Leitung (24) und (8) in die Verbrennungsanlage (9). Nach Zeitprogramm gesteuert schließt Dampfregelventil (19) und die Aktiv-Kohle im Adsorber (10) wird mit dem Abgas auf dem gleichen Wege wie eben beschrieben in der 3. Regenerierungstufe abgekühlt, bevor ebenfalls zeitgesteuert die beiden Vierwegeventil (5) und (7) auf den anderen Adsorber umschalten und der Vorgang von neuem beginnt.

Beispiel 1

In einen mit Deacon-Katalysator (z.B. $CuCl_2/Al_2O_3$) gefüllten Fließbettreaktor wird $C_2H_4$, HCl und $O_2$ im Verhältnis von etwa 1 : 1,9 : 0,6 eingespeist und bei 220° C und 4 bar zur Reaktion gebracht. Dabei entsteht durch Oxichlorierung hauptsächlich 1,2-Dichlorethan und Wasser. $C_2H_4$ und $O_2$ werden in Überschuß angewendet, um HCl möglichst vollständig umzusetzen und so Korrosion an der Apparatur zu vermeiden.

Nach Verlassen des Reaktorraumes wird das Reaktionsgas in einer 1. Kondensationsstufe durch Einspritzen von gegebenenfalls alkalisiertem Wasser auf 90° C abgekühlt. Danach wird das Reaktionsgas in 2 Kondensationsstufen, zuerst mit Wasser auf 38° C, dann mit Sole auf 7° C, abgekühlt und dabei 1,2-Dichlorethan und Wasser auskondensiert.

Das hinter der 3. Kondensationsstufe mit 7° C austretende Gas enthält $N_2$, das in geringer Menge mit den Ausgangsgasen und zu Spül- und Meßzwecken in den Reaktor eingespeist wird und sich allmählich anreichert, sowie CO und $CO_2$, die bei der Reaktion als Nebenprodukte durch Verbrennung von $C_2H_4$ entstehen und sich ebenfalls anreichern, bei ein Gleichgewicht zwischen Neubildung und Ausschleusung erreicht ist. Das Gas enthält weiter 0,37 Vol% $C_2H_4$ und 5,9 Vol% $O_2$ entsprechend dem angewandten Überschuß und < 1 Vol% leichtsiedende organische Nebenprodukte. Außerdem ist es entsprechend dem Druck und der Temperatur mit 1,2-Dichlorethan und Wasser gesättigt.

Läßt man den Wassergehalt außer Betracht, so hat das Reaktiongas folgende quantitative Zusammensetzung in Vol%:

| | vor dem Adsorber | hinter dem Adsorber |
|---|---|---|
| $H_2$ | 0,21 | 0,23 |
| Ar | 2,8 | 2,8 |
| $O_2$ | 5,9 | 6,1 |
| $N_2$ | 53,9 | 53,9 |
| $CH_4$ | 0,036 | 0,037 |
| CO | 2,9 | 2,9 |
| $CO_2$ | 32,5 | 33,3 |
| $C_2H_4$ | 0,37 | 0,42 |
| $C_2H_6$ | 0,23 | 0,24 |
| $CH_3Cl + CH_2=CHCl$ | 0,38 | 0,02 |
| $C_2H_5Cl + CH_3CHO$ | < 0,02 | < 0,02 |
| 1,2-Dichlorethan | 1,0 | < 1    ppm |

Es wird auf ca. 6 bar komprimiert, mit $O_2$ auf 20 Vol% $O_2$ angereichert und wieder in den

Oxichlorierungsreaktor eingespeist, wo $O_2$ erneut reagiert.

Vor dem Komprimieren wird über eine Druckregeleinrichtung, die den Druck im Reaktor und Gaskreislauf konstant hält, genau die Menge als Abgas ausgeschleust, die dem Zugang an Inertgas und an gasförmigen Nebenprodukten entspricht. Im vorliegenden Beispiel sind dies 900 $m^3$/h Abgas, entsprechend 70 $m^3$ Abgas je t 1,2-Dichlorethan, gerechnet unter Normalbedingungen (0°C, 1,013 bar).

In einer Aufheizstrecke wird dieses Abgas von 7°C und 1,7 bar auf 30°C, d.h. 23°C über dem Taupunkt des Wassers, erwärmt und dadurch die Adsorptionskapazität der Aktivkohle für 1,2-Dichlorethan optimiert. Dann wird das Abgas durch einen der beiden mit Aktivkohle mit einer BET-Oberfläche von 1400 $m^2$/g gefüllten zylindrischen Adsorber (Höhe 7,0 m; Durchmesser 0,9 m; Rauminhalt 4,45 $m^3$; Verhältnis Höhe : Durchmesser = 7,7 : 1) geleitet und dabei das 1,2-Dichlorethan vollständig bis auf < 1 ppm entfernt. Wie die Gasanalyse zeigt, werden auch Methylchlorid und Vinylchlorid von der Aktivkohle adsorbiert und mit Wasserdampf wieder desorbiert, doch ist ihre Rückgewinnung ohne Interesse.

Danach wird das Abgas direkt oder, wenn für die Regenerierung benötigt, über den 2. Adsorber zur Weiterbehandlung in eine Verbrennungsanlage geleitet. In einer Verbrennungsmuffel werden bei einer Temperatur > 900°C die restlichen organischen Bestandteile vernichtet und in nachgeschalteten Wäschen HCl und Chlor entfernt, bevor das Abgas in die Atmosphäre austritt.

Als Adsorber werden 2 gleichartige Behälter verwendet, deren Inhaltsvolumen so ausgelegt ist, daß die Adsorptionskapazität eines Adsorbers für 1,2-Dichlorethan zeitlich ausreicht, um währenddessen den anderen Adsorber zu regenerieren. Die Umschaltung vom adsorbierenden zum regenerierenden Adsorber erfolgt automatisch zeitprogrammgesteuert im 12 Stunden-Intervall mittels 2 Vierwege-Ventilen. Jeweils eine der Adsorber befindet sich also in Betrieb, während der andere in 3 Stufen regeneriert wird.

1. Stufe: Desorbieren des 1,2-Dichlorethans mit Dampf von 8 bar. 1,2-Dichlorethan und Dampf werden auskondensiert und laufen in einen Abscheider ab, wo sie sich wegen ihrer Unlöslichkeit ineinander und ihrer unterschiedlichen Dichte trennen. Das spezifisch schwere 1,2-Dichlorethan bildet die untere Phase und wird daher aufgrund der Standregelung im Abscheider sofort in die Oxichlorierungsanlage zur Aufarbeitung zurückgepumpt, gefolgt, von einem Teil der oberen Phase, dem Wasser.

2. Stufe: Trocknen der Aktivkohle mit 900 $m^3$/h (Normalbedingungen) auf 124°C erhitztem, vorher von 1,2-Dichlorethan befreitem Abgas, das durch den Adsorber, den Kondensator und den Abscheider zur Verbrennungsanlage strömt.

3. Stufe: Kaltblasen mit dem nicht erhitzten Abgas (900 m3/h unter Normalbedingungen, 30°C) wie unter Stufe 2 beschrieben.

Sämtliche für das Regenerieren und Umschalten der Adsorber erforderlichen Funktionen erfolgen automatisch von einem Zeitprogramm gesteuert.

Beispiel 2 (vergleichsbeispiel)

Man verfährt gemäß Beispiel 1, jedoch werden nur 860 $m^3$/h Abgas, entsprechend 68 $m^3$ Abgas je t 1,2-Dichlorethan, gerechnet unter Normalbedingungen, aus dem Gaskreislauf der Oxichlorierungsanlage ausgeschleust. Auf eine Erwärmung des bei 7°C und 1,7 bar ausgeschleusten Abgases wird verzichtet, so daß die Adsorption an Aktivkohle bei 7°C erfolgt. Das 1,2-Dichlorethan wird nur bis auf 0,13 Vol% entfernt, wie folgende Gasanalyse zeigt (Vol%):

|  | vor dem Adsorber | hinter dem Adsorber |
|---|---|---|
| $H_2$ | 0,24 | 0,24 |
| Ar | 2,90 | 2,90 |
| $O_2$ | 6,00 | 6,00 |
| $N_2$ | 55,00 | 55,10 |
| $CH_4$ | 0,04 | 0,04 |
| CO | 3,0 | 3,0 |
| $CO_2$ | 33,2 | 33,4 |
| $C_2H_4$ | 0,51 | 0,50 |
| $C_2H_6$ | 0,21 | 0,21 |
| $CH_3Cl + CH_2=CHCl$ | 0,31 | 0,04 |
| $C_2H_5Cl + CH_3CHO$ | < 0,02 | < 0,02 |
| 1,2-Dichlorethan | 1,0 | 0,13 |

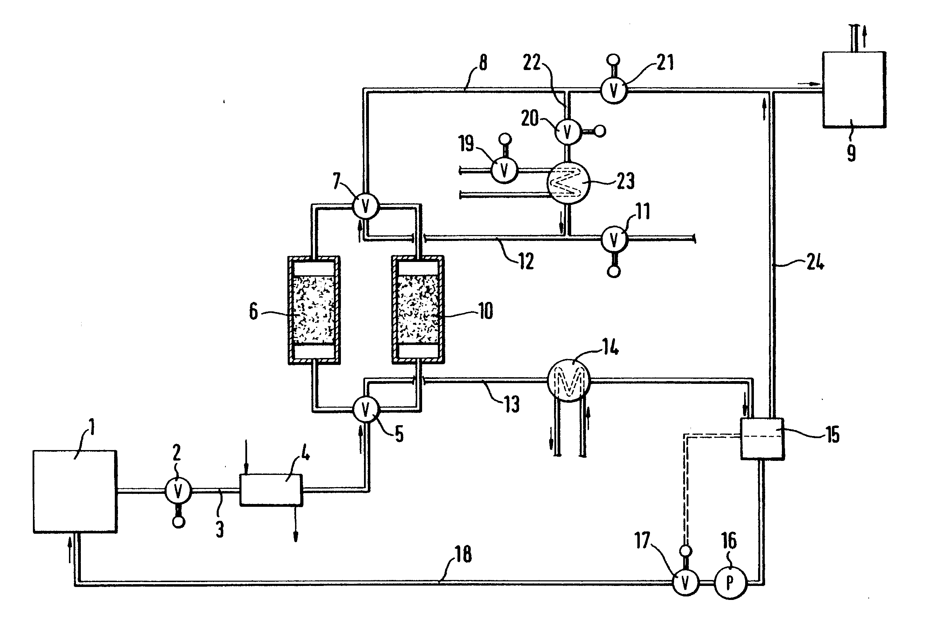

## Ansprüche

1. Kontinuierliches Verfahren zur vollständigen Entfernung und Rückgewinnung von 1,2-Dichlorethan aus den bei der Herstellung von 1,2-Dichlorethan durch Oxichlorierung von Ethylen mit Chlorwasserstoff und Sauerstoff anfallenden Abgasen, welche über Aktivkohle geleitet und anschließend bei Temperaturen oberhalb 900° C verbrannt sowie schließlich von Chlorwasserstoff und Chlor gereinigt werden, wobei die Aktivkohle mit heißem Wasserdampf von 1,2-Dichlorethan befreit und regeneriert wird, dadurch gekennzeichnet, daß man das mit 1,2-Dichlorethan beladene, eine Temperaturs von 1° bis 10° C aufweisende Abgas auf eine Temperatur, die mindestens 20° C oberhalb des jeweiligen Taupunktes für Wasser liegt, erwärmt und über die Aktivkohle leitet, welche in zwei gleichartigen Zonen angeordnet ist, wovon in einem Zeitraum von 8 bis 16 Stunden jeweils eine Zone 1,2-Dichlorethan aus dem Abgas adsorbiert, während die andere Zone in drei Stufen regeneriert wird, derart, daß man die mit 1,2-Dichlorethan beschickte Aktivkohle-Zone in der ersten Regenerierungsstufe mit Wasserdampf von 4 bis 10 bar beaufschlagt und von 1,2-Dichlorethan befreit, in der zweiten Regenerierungsstufe mit 1,2-dichlorethanfreiem und auf 100 bis 150° C erhitztem Abgas trocknet, in der dritten Regenerierungsstufe mit 1,2-dichlorethanfreiem Abgas kaltbläst, kondensiertes 1,2-Dichlorethan und Dampfkondensat in die Oxichlorierung zurückführt und das Abgas der Verbrennung zuführt.

2. Verfahren nach Anspruch 1, dadurch gekennzeichnet, daß man das mit 1,2-Dichlorethan beladene Abgas auf 25 bis 35° C erwärmt.

3. Verfahren nach Anspruch 1 oder 2, dadurch gekennzeich net, daß man eine Aktivkohle mit einer aktiven Oberfläche nach BET von mindestens 1200 $m^2/g$ einsetzt.

4. Verfahren nach mindestens einem der Ansprüche 1 bis 3, dadurch gekennzeichnet, daß die Verweilzeit des Abgases in der 1,2-Dichlorethan adsorbierenden Aktivkohle-Zone mindestens 10 Sekunden beträgt.

5. Verfahren nach mindestens einem der Ansprüche 1 bis 4, dadurch gekennzeichnet, daß das Umschalten von einer Regenerierungsstufe auf die nächste und von einer auf die andere Aktivkohlezone vollautomatisch von einem Zeitprogramm gesteuert wird.

EP 0 255 893 B1

6. Vorrichtung zur kontinuierlichen Durchführung des Verfahrens gemäß mindestens einem der Ansprüche 1-5, ge-kennzeichnet durch 2 gleichartige, senkrecht angeordnete, jeweils oben und unten durch Verbindungsleitungen überbrückte, mit Aktivkohle gefüllte zylindrische Adsorber (6, 10), deren Verhältnis Höhe zu Durchmesser 5 : 1 bis 10 : 1 beträgt, wobei die Adsorptionskapazität der Aktivkohlemenge eines Adsorbers zeitlich ausreicht für die Regenerierung der gleichen Aktivkohlemenge im anderen Adsorber; je 1 Vierwegeventil (5, 7) in den beiden Verbindungsleitungen; eine erste Zuführleitung (3) für 1,2-dichlorethanhaltiges Abgas von der Oxichlorierungsanlage (1) zum unteren Vierwegeventil (5), eine Heizung (4) in der Zuführleitung (3); eine erste Abzugsleitung (8) für 1,2-dichlorethanfreies Abgas vom oberen Vierwegeventil (7) zu einem Verbrennungsofen (9); eine zweite Zuführleitung (12) mit Absperrventil (11) zum oberen Vierwegeventil (7) zwecks Transport von Heißdampf für die abwechselnde Desorption der Aktivkohle sowie von 1,2-dichlorethanfreiem Abgas zum Trocknen oder Kaltblasen der desorbierten Aktivkohle; eine dritte Verbindungsleitung (22) für 1,2-dichlorethanfreies Abgas mit Wärmetauscher (23) zwischen der ersten Abzugsleitung (8) und der zweiten Zuführleitung (12); eine zweite Abzugsleitung (13) für 1,2-dichlorethanhaltigen Heißdampf sowie wasserdampfgesättigtes Abgas mit Kondensator (14) vom unteren Vierwegeventil (5) zu einem Abscheider (15) für die Abscheidung und gleichzeitige Phasentrennung von kondensiertem 1,2-Dichlorethan und Wasser.

7. Vorrichtung nach Anspruch 6, gekennzeichnet durch eine dritte Abzugsleitung (24) für 1,2-dichlorethanfreies Abgas vom Abscheider (15) zum Verbrennungsofen (9).

8. Vorrichtung nach Anspruch 6 oder 7, gekennzeichnet durch eine vierte Abzugsleitung (18) für 1,2-Dichlorethan und Kondensat vom Abscheider (15) zur Oxichlorierungsanlage (1) mit Standhaltungsregler (17) für den Abscheider (15).

## Claims

1. A continuous process for the complete removal and recovery of l,2-dichloroethane from the waste gases arising in the production of 1,2-dichloroethane by oxychlorination of ethylene with hydrogen chloride and oxygen, the waste gases being passed over activated carbon and then being burned at temperatures above 900° C and finally being purified by removal of hydrogen chloride and chlorine, the activated carbon being freed of l,2-dichloroethane by means of superheated steam and regenerated, which comprises heating the waste gas, which is laden with l,2-dichloroethane and is at a temperature from 1° to 10° C, to a temperature which is at least 20° c above the particular dew point for water and passing it over the activated carbon which is arranged in two identical zones, of which one adsorbs 1,2-dichloroethane from the waste gas over a period of from 8 to 16 hours in each case, while the other zone is being regenerated in three stages, in such a way that the activated carbon zone charged with 1,2-dichloroethane is subjected in the first regeneration stage to steam of 4 to 10 bar and freed of 1,2-dichloroethane, is dried in the second regeneration stage by means of waste gas free of 1,2-dichloroethane and heated to 100-150° C, blown cold in the third regeneration stage with waste gas free of 1,2-dichloroethane, condensed 1,2-dichloroethane and steam condensate are recycled to the oxychlorination and the waste gas is fed to the combustion.

2. The process as claimed in claim 1, wherein the waste gas laden with 1,2-dichloroethane is heated to 25-35° C.

3. The process as claimed in claim 1 or 2, wherein an activated carbon having an active BET surface area of at least 1200 m²/g is used.

4. The process as claimed in at least one of claims 1 to 3, wherein the residence time of the waste gas in the activated carbon zone adsorbing 1,2-dichloroethane is at least 10 seconds.

5. The process as claimed in at least one of claims 1 to 4, wherein the change-over from one regeneration stage to the next and from one activated carbon zone to the other is controlled fully automatically by a time program.

6. Equipment for continuously carrying out the process according to at least one of claims 1-5, which comprises 2 identical, vertically arranged cylindrical adsorbers (6, 10) which are bridged by connecting

7

lines both at the top and bottom and are charged with activated carbon and whose height : diameter ratio is 5 : 1 to 10 : 1, the adsorption capacity of the quantity of activated carbon of one adsorber allowing sufficient time for the regeneration of the same quantity of activated carbon in the other adsorber, 1 four-way valve (5, 7) in each of the two connecting lines, a first feed line (3) for waste gas containing 1,2-dichloroethane from the oxychlorination plant (1) to the lower four-way valve (5), a heater (4) in the feed line (3), a first take-off line (8) for waste gas free of 1,2-dichloroethane, leading from the upper four-way valve (7) to a combustion furnace (9), a second feed line (12) having an isolation valve (11) and leading to the upper four-way valve (7) for transporting superheated steam for the alternating desorption of the activated carbon and waste gas free of 1,2-dichloroethane for drying or cold-blowing the desorbed activated carbon, a third connecting line (22) for waste gas free of 1,2-dichloroethane with a heat exchanger (23) between the first take-off line (8) and the second feed line (12), a second take-off line (13) for superheated steam containing 1,2-dichloroethane and for steam-saturated waste gas, having a condenser (14) and leading from the lower four-way valve (5) to a precipitator (15) for the precipitation and simultaneous phase separation of condensed l,2-dichloroethane and water.

7. Equipment as claimed in claim 6, which comprises a third take-off line (24) for waste gas free of 1,2-dichloroethane, leading from the precipitator (15) to the combustion furnace (9).

8. Equipment as claimed in claim 6 or 7, which comprises a fourth take-off line (18) for 1,2-dichloroethane and condensate, leading from the precipitator (15) to the oxychlorination plant (1) and having a level controller (17) for the precipitator (15).

**Revendications**

1. Procédé pour la séparation complète et la récupération de 1,2-dichloroéthane des gaz résiduaires se formant dans la fabrication de 1,2-dichloroéthane par oxychloration de l'éthylène par le chlorure d'hydrogène et l'oxygène, qui sont envoyés sur du charbon actif et ensuite brûlés à des températures de plus de 900°C et enfin purifiés par séparation du chlorure d'hydrogène et du chlore, le charbon actif étant débarrassé du 1,2-dichloroéthane par la vapeur d'eau chaude et régénéré caractérisé en ce que l'on chauffe le gaz résiduaire, d'une température de 1-10°C, chargé de 1,2-dichloroéthane à une température qui est d'au moins 20°C au-dessus du point de rosée correspondant de l'eau et on l'envoie sur du charbon actif qui est disposé dans deux zones de même type, dont chaque fois en 8 à 16 h, une zone adsorbe le 1,2-dichloroéthane du gaz résiduaire, tandis que l'autre zone est régénérée en trois étapes, dans lesquelles dans la première étape de régénération, on charge la zone de charbon actif chargée de 1,2-dichloroéthane avec de la vapeur d'eau sous 4-10 bar et on la débarrasse du 1,2-dichloroéthane, dans la seconde étape de régénération, on la sèche par du gaz résiduaire exempt de 1,2-dichloroéthane et chauffé à 100-150°C et dans la troisième étape de régénération, on la refroidit par soufflage de gaz résiduaire exempt de 1,2-dichloroéthane, on renvoie dans l'oxychloration le 1,2-dichloroéthane condensé et le condensat de vapeur d'eau et on envoie le gaz résiduaire à la combustion.

2. Procédé selon la revendication 1, caractérisé en ce que l'on chauffe le gaz résiduaire chargé en 1,2-dichloroéthane à 25-35°C.

3. Procédé selon la revendication 1 ou 2, caractérisé en ce que l'on utilise un charbon actif ayant une surface spécifique selon BET d'au moins 1 200 m²/g.

4. Procédé selon l'une au moins des revendications 1 à 3, caractérisé en ce que la durée de passage du gaz résiduaire dans la zone de charbon actif adsorbant le 1,2-dichloroéthane est d'au moins 10 s.

5. Procédé selon l'une au moins des revendications 1 à 4, caractérisé en ce que le changement d'une étape de régénération à la suivante et d'une zone de charbon actif à l'autre s'effectue par régulation à programme entièrement automatique.

6. Appareil pour la mise en oeuvre continue du procédé selon l'une au moins des revendications 1 à 5, caractérisé par 2 adsorbeurs cylindriques de même type (6, 10) disposés verticalement, raccordés chaque fois en haut et en bas par des conduites de liaison, remplis de charbon actif, dont le rapport de la hauteur au diamètre est de 5:1 à 10:1, la capacité d'adsorption de la quantité de charbon actif d'un adsorbeur étant suffisante dans le temps pour la régénération de la même quantité de charbon actif dans l'autre adsorbeur ; une vanne à quatre voies (5, 7) dans ohacune des deux conduites de liaison, une première conduite d'amenée (3) pour le gaz résiduaire contenant le 1,2-dichLoroéthane de l'installation d'oxychloration (1) à la vanne à quatre voies inférieure (5), un chauffage (4) dans la conduite d'amenée (3) ; une première conduite d'évacuation (8) pour le gaz résiduaire exempt de 1,2-dichloroéthane de la vanne à quatre voies supérieure (7) à un four de combustion (9) ; une seconde conduite d'amenée (12) avec soupape d'arrêt (11) à la vanne à quatre voies supérieure (7) pour le transport de vapeur chaude pour la description alternée du charbon actif ainsi que de gaz résiduaire exempt de 1,2-dichLoroéthane pour le séchage ou le refroidissement par soufflage du charbon actif désorbé ; une troisième conduite de liaison (22) pour le gaz résiduaire exempt de 1,2-dichloroéthane avec échangeur de chaleur (23) entre la première conduite d'évacuation (8) et la seconde conduite d'amenée (12), une seconde conduite d'évacuation (13) pour la vapeur d'eau chaude contenant du 1,2-dichloroéthane et le gaz résiduaire saturé de vapeur d'eau avec un condenseur (14) de la vanne à quatre voies inférieure (5) à un séparateur (15) pour la collecte et la séparation de phases simultanées du 1,2-dichloroéthane condensé et de l'eau.

7. Appareil selon la revendication 6, caractérisé par une troisième conduite d'évacuation (24) pour le gaz résiduaire exempt de 1,2-dichloroéthane du séparateur (15) au four de combustion (9).

8. Appareil selon la revendication 6 ou 7, caractérisé par une quatrième conduite d'évacuation (18) pour le 1,2-dichloroéthane et le condensat du séparateur (15) à l'installation d'oxychloration (1) avec un régulateur de niveau (17) pour le séparateur (15).

EP 0 255 893 B1